# EUROPÄISCHE PATENTSCHRIFT

(11) **EP 0 952 137 B1**
(45) Veröffentlichungstag und Bekanntmachung des Hinweises auf die Patenterteilung: **21.07.2004**
(21) Anmeldenummer: 99107633.2
(22) Anmeldetag: 16.04.1999
(51) Int. Cl.: C07C 39/08, C07C 37/07

(54) **Neues Verfahren zur Herstellung von 2,3,5-Trimethylhydrochinondiestern**
Process for the preparation of 2,3,5-trimethylhydroquinone diester
Procédé pour la préparation de diesters de 2,3,5-trimethylhydroquinone

(30) Priorität: 21.04.1998 DE 19817644
(43) Veröffentlichungstag der Anmeldung: 27.10.1999
(73) Patentinhaber: Degussa AG, 40474 Düsseldorf (DE)
(72) Erfinder: Shi, Nongyuan, Dr., 63512 Hainburg (DE); Scholz, Mario, Dr., 63584 Gründau (DE); Hasenzahl, Steffen, Dr., 63477 Maintal (DE); Weigel, Horst, 63517 Rodenbach (DE); Drapal, Bernd, 63755 Alzenau (DE); McIntosh, Ralph, 63457 Hanau (DE); Hasselbach, Hans Joachim, Dr., 63571 Gelnhausen (DE); Huthmacher, Klaus, Dr., 63584 Gelnhausen (DE)

(56) Entgegenhaltungen:
- EP-A- 0 850 910
- DE-A- 2 149 159
- DE-A- 2 646 172
- DE-A- 19 627 977

## Beschreibung

Die Erfindung betrifft ein neues Verfahren zur Herstellung von 2,3,5-Trimethylhydrochinondiestern durch Umlagerung von 2,6,6-Trimethylcyclohex-2-en-1,4-dion (4-Oxo-Isophoron, Ketoisophoron) in Gegenwart eines festen, sauren Katalysators und eines Acylierungsmittels, wie z. B. Cabonsäureanhydriden, Carbonsäurehalogeniden. Der 2,3,5-Trimethylhydrochinondiester kann gegebenenfalls anschließend zum freien 2,3,5-Trimethylhydrochinon verseift werden, das ein wertvoller Baustein bei der Synthese von Vitamin E ist.

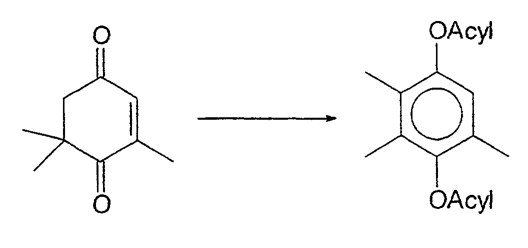

### Stand der Technik

2,3,5-Trimethylhydrochinon (TMHQ) ist ein wichtiges Zwischenprodukt, das bei der Produktion von Vitamin-E bzw. Vitamin-E-Acetat verwendet wird. Neben den bekannten Herstellungsverfahren aus aromatischen Ausgangsmaterialien kann 2,3,5-Trimethylhydrochinon aus einer nichtaromatischen Verbindung, dem 2,6,6-Trimethylcyclohex-2-en-1,4-dion, durch Umlagerung unter acylierenden Bedingungen und anschließender Hydrolyse hergestellt werden.

In der Patentschrift DE 26 46 172 C2 wird ein Verfahren beschrieben, bei dem 2,6,6-Trimethylcyclo-hex-2-en-1,4-dion in der Dampfphase bei höher Temperatur in Kontakt mit einem sauren Katalysator zu Trimethylhydrochinon direkt umgelagert wird. Allerdings ist die Ausbeute bei diesem Verfahren nur gering (50% bei 30% Umsatz). Wird die Aromatisierung von 2,6,6-Trimethylcyclohex-2-en-1,4-dion in Gegenwart eines Acylierungsmittels durchgeführt, so werden Trimethylhydrochinon-diester erhalten, die durch anschließende Hydrolyse zum Trimethylhydrochinon führen.
So wird beispielsweise gemäß *Bull. Korean. Chem. Soc*. 1991, *12*, 253 die Umlagerung in 5 %-iger Lösung in Acetanhydrid durch Zugabe von fünf Äquivalenten konzentrierter Schwefelsäure durchgeführt. Der Trimethylhydrochinondiester wird dabei lediglich mit 30% Ausbeute erhalten.

In einem weiteren Verfahren gemäß DE-OS 2 149 159 kann 2,6,6-Trimethylcyclohex-2-en-1,4-dion in Gegenwart von Acetanhydrid in einer durch Protonen- oder Lewissäurenkatalysierten Umlagerung zu Trimethylhydrochinondiacetat umgewandelt werden, welches anschließend zu Trimethylhydrochinon verseift wird. Mäßige bis gute Ausbeuten werden bei diesem Verfahren genannt.

Aus der DE-OS 196 27977 ist bekannt, TMHQ durch Umsetzung von Ketoisophoron mit einem Acylierungsmittel in Gegenwart sehr starker Säuren, wie z. B. Fluorsulfonsäure oder Oleum herzustellen. Bei diesem Verfahren folgt auf die zunächst eintretende Bildung des entsprechenden Esters die Verseifung.

EP850 910 beschreibt ein Verfahren zur Herstellung von Trimethylhydrochinondiestern in Gegenwart eines Festkatalysators.

Die bekannten Verfahren haben den Nachteil, daß entweder die Ausbeuten niedrig sind oder daß gleichzeitig durch den Einsatz von starken Säuren in gelöster Form Korrosionsprobleme auftreten, die den Einsatz von hochwertigen Werkstoffen erforderlich machen. Die Abtrennung und Recyclierung des gelösten Katalysators sind außerdem nur schwierig durchzuführen.

### Aufgabe

Die Aufgabe besteht darin, ein Verfahren zur Herstellung von 2,6,6-Trimethylcyclohex-2-en-1,4-dion-diestern zu finden, das die Nachteile der bekannten Verfahren überwindet. Aus den Estern sind gegebenfalls die entsprechenden Hydrochinone durch Hydrolyse zu gewinnen.

### Lösung der Aufgabe

Es wurde gefunden, daß 2,6,6-Trimethylcyclohex-2-en-1,4-dion in Gegenwart einer festen Säure mit einem Acylierungsmittel zu einem Trimethylhydrochinondiester umgesetzt wird. Durch eine sich gegebenenfalls anschließende Verseifung erhält man 2,3,6-Trimethylhydrochinon.

Für das Verfahren können prinzipiell alle als Säuren wirkende Feststoffe eingesetzt werden, die unter den Reaktionsbedingungen stabil sind. Beispiele hierfür sind kristalline und/oder amorphe Alumosilikate, Tonmineralien oder Pillard Clays die jeweils in der H-Form eingesetzt werden, Mineralsäuren auf geeigneten Trägern wie z.B. Schwefelsäure auf ZrO₂ oder SnO₂ bzw. Phosphorsäure auf SiO₂, Ionenaustascherharze mit sauren Gruppen, insbesondere auf fluorierter Basis wie Nafion-H® (Du Pont) oder Amberlyst® (Rohm und Haas) sowie Polysiloxane mit sauren Gruppen wie z.B. Deloxan ASP® (Degussa). Als saure Gruppen dienen insbesondere SO₃-Gruppen.

Besonders geeignet sind saure, großporige (mit 12-Ringporen) Zeolithe mit Porendurchmessern zwischen 0.5 und 0.8 nm. Beispiele hierfür sind Y-Zeolithe, Beta-Zeolithe, dealuminierte Zeolithe oder Mordenite. Diese werden besonders in Atlas of Zeolite Structure Types" (W.M. Meier et al., 4^{th} Revised Edition, Elsevier, 1996) beschrieben. Prinzipiell sind saure Zeolithtypen mit dem genannten oder größeren Porendurchmessern geeignet. Auch geeignet sind mittelporige (mit 10-Ringporen) Zeolithe, z.B. vom ZSM-5-Typ.

Der Modul, das molare SiO₂/Al₂O₃-Verhältnis eines Zeoliths, das ein wichtiges Maß für dessen Säurekapazitat ist, kann in weiten Grenzen variiert werden. Der Modul eines gegebenen Zeolithtyps kann im wesentlichen durch die Zusammensetzung des Synthesegels, aus dem er kristallisiert wird, bestimmt werden. Im Fall der Y-Zeolithe kann dieser auch durch die nachträgliche Dealuminierung, beispielsweise durch Umsetzung mit Wasserdampf oder SiCl₄, in einem weitem Bereich eingestellt werden. Übliche Zeolithsynthesen, wie sie beispielsweise in Handbook of Molecular Sieves" (R. Szostak, Van Nastrand Renhold, 1992) und darin zitierter Literatur beschrieben werden, liefern die Zeolithe im allgemeinen in der katalytisch inaktiven Na-Form. Um sie in die katalytisch aktive H-Form überzuführen, kann ein Ionenaustausch mit Säuren wie z.B. Salzsäure oder Schwefelsäure oder mit Ammoniumsalzen wie z.B. NH₄Cl, (NH₄)₂SO₄ oder NH₄-Acetat und einer anschließenden Kalzination durchgeführt werden.

Für das erfindungsgemäße Verfahren eignen sich vor allem HY-Zeolithe mit einem Modul zwischen 7,5 und 200, insbesondere zwischen 25 und 120, H-Beta-Zeolithe mit einem Modul zwischen 13 und 60, insbesondere zwischen 18 und 30 und H-Mordenite mit einem Modul zwischen 5 und 100, vor allem zwischen 10 und 30.

Ebenfalls gut für das erfindungsgemäße Verfahren geeignete Materialien sind die in den letzten Jahren entdeckten Alumosilikate mit regelmäßigen Mesoporenstruktur, wie z.B. MCM-41 oder MCM-48. Hier ermöglichen die Mesoporen mit Porendurchmessern zwischen 2.0 und 10.0 nm eine rasche Diffusion der Reaktanden zu den katalytisch aktiven Zentren.

Die Zeolithe oder Alumosilikate mit regelmäßiger Mesoporenstruktur können im erfindungsgemaßen Verfahren in verformter oder unverformter Form eingesetzt werden. Die unverformten Materialien erhält man direkt nach der Synthese und einem eventuellen Ionenaustausch. Die Formgebung kann im Anschluß an die Synthese mittels bekannter Verfahren wie zum Beispiel der Granulation, z.B. durch Sprühtrocknung, Wirbelschicht-Sprühgranulationstrocknung oder die Tellergranulation, die Extrusion sowie die Tablettierung erfolgen. Beispiele für mögliche Binder, die bei dem Formgebungsschritt zugesetzt werden können, sind Siliciumdioxid, Aluminiumoxid, Titandioxid und Tonmineralien. Für das erfindungsgemäße Verfahren kommt insbesondere der Einsatz von Formkörpern bei Festbettfahrweise oder von Granulaten bei Suspensionfahrweise in Frage.

Die als Katalysatoren eingesetzten Materialien verlieren im allgemeinen während der Reaktion an katalytischer Aktivität. Ursache hierfür ist vor allem die Ablagerung von höhermolekularen Folge- oder Nebenprodukten im Porensystem. Um die ursprüngliche Aktivität wiederherzustellen, müssen diese durch geeignete Verfahren entfernt werden. Dies kann bei anorganischen Materialien beispielsweise durch Kalzination in einem Muffelofen, einem Drehrohr oder einem anderen geeigneten Aggregat bei einer Temperatur zwischen 250 und 800°C, bevorzugt zwischen 400 und 650°C, geschehen. Die Kalzination erfolgt dabei im allgemeinen in einer Luftoder Inertgas-Atmosphäre. Besonders vorteilhaft ist es, die Kalzination zunächst in Stickstoff-Atmosphäre und daran anschließend in Luftatmosphäre durchzuführen. Die Kalzinationsdauer ist den Umständen leicht anzupassen, wobei im allgemeinen eine Dauer von 2 h ausreicht. Die Aufheizrate ist in einem weiten Bereich variierbar. Haben sich keine oder nur geringfügige Mengen höhermolekularer Produkte gebildet, kann die Regenerierung auch mit Hilfe einer Extraktion mit geeigneten Lösungsmitteln durchgeführt werden. Hier eignen sich beispielsweise Ester, wie z.B. Ethylacetat; Ketone, wie z.B. Aceton; organischen Säuren, wie z.B. Essigsäure, Säureanhydride, z.B. Essigsäureanhydrid oder Alkohole. In diesem Fall wird der zu regenerierende Katalysator mit dem entsprechenden Lösungsmittel bei Raumtemperatur oder erhöhter Temperatur gerührt. Der Feststoff wird dann abgetrennt, beispielsweise durch Filtration oder Zentrifugation, eventuell getrocknet, kalziniert und wieder in das Verfahren zurückgeführt.

Neben Zeolithen und Alumosilikaten sind weitere feste Säuren, die unter den acylierenden Bedingungen stabil sind, geeignet. Beispiele hierfür sind Mineralsäuren auf geeigneten Trägern, aber auch Polymere die stark saure Gruppen enthalten. Bevorzugt wird aus dieser Gruppe ein perfluoriertes Sulfonsäuregruppen-haltiges Polymerisat, Nafion-H® der Firma DuPont, das thermisch und chemisch besonders resistent ist. Besonders bevorzugt ist hierbei eine Modifikation mit großer Oberfläche, die durch Silikonvernetzung entsteht (M. A. Harmer, *J. Am. Chem. Soc*., 118, 1996, 7709 ).

Die Einsatzmenge des Katalysators liegt zwischen 5 und 150 Gew. % bezogen auf 2,6,6-Trimethylcyclohex-2-en-1,4-dion, bevorzugt zwischen 20 und 60 Gew% bezogen auf 2,6,6-Trimethylcyclohex-2-en-1,4-dion.

Die Umlagerung erfolgt zweckmäßigerweise bei Temperaturen zwischen etwa 0°C und 140°C, vorzugsweise zwischen etwa 20°C und 100°C.

Bei dem erfindungsgemäß verwendeten Acylierungsmittel handelt es sich vorzugsweise um ein Carbonsäureanhydrid, einen Enolester oder ein Carbonsäurechlorid. Insbesondere wird ein Carbonsäureanhydrid der allgemeinen Formel I verwendet, 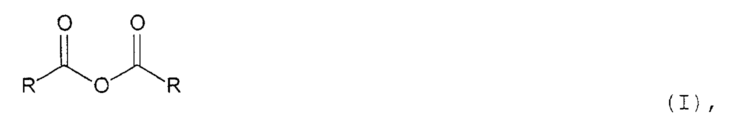 in der R einen gegebenenfalls substituierten aliphatischen, alicyclischen oder aromatischen Rest mit 1-8 Kohlenstoffatomen, der gegebenenfalls 1 bis 3 Halogenatome enthält, bedeutet. Geeignet sind insbesondere die Anhydride von Essigsäure, Propionsäure, Buttersäure, Isobuttersäure Cyclohexancarbonsäure, Benzoesäure, Chloressigsäure. Besonders geeignet ist Acetanhydrid.
Anstelle der Säureanhydride können auch andere Acylierungsmittel, wie z. B. Enolester oder Acylhalogenide eingesetzt werden.

Beispiele für geeignete Acylhalogenide sind Acetylchlorid, Propionsäure-, Buttersäurechlorid.Enolester, wie z. B. Vinylacetat, Isopropenylacetat und Isopropenylisobutyrat dienen als Acylierungsmittel in Gegenwart von Katalysatoren und eignen sich speziell zur Durchführung des beanspruchten Verfahrens. Als Acylierungsmittel geeignete Enolester entsprechen der allgemeinen Formel, 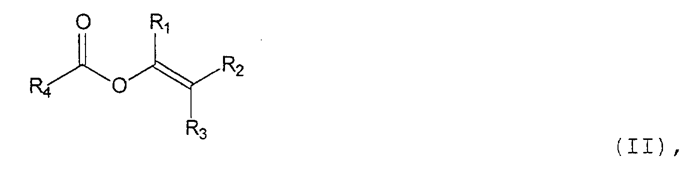 in der R₁ und R₂ Wasserstoffatome oder Alkylreste mit 1 bis 8 Kohlenstoffatomen oder Alkylenreste mit 1 bis 5 Kohlenstoffatomen, die gemeinsam einen 5- oder 6-gliedrigen Kohlenstoffring bilden, R₃ ein Wasserstoffatom oder einen Alkylrest mit 1 bis 8 Kohlenstoffatomen und R₄ einen aliphatischen, oder aromatischen Kohlenwasserstoffrest mit 1 bis zu 8 Kohlenstoffatomen bedeutet.

Das Molverhältnis zwischen dem Acylierungsmittel und 2,6,6-Trimethylcyclohex-2-en-1,4-dion ist variabel. Bei einer Umsetzung ohne zusätzlichem Lösungsmittel soll das Molverhältnis von Acylierungsmittel zu 2,6,6-Trimethylcyclohex-2-en-1,4-dion zwischen 3 : 1 und 10 : 1, vorzugsweise zwischen 3 : 1 und 5 : 1 liegen. Das überschüssige Acylierungsmittel dient als Lösungsmittel das nach der Abtrennung des festen Katalysators destillativ zurückgewonnen werden kann.

Die Umlagerung erfolgt ebenso in Gegenwart organischer Lösungsmittel. Als geeignete Lösungsmittel dienen aliphatische und cyclische Ester, z. B. Essigsäureethylester, Essigsäurepropylester, Essigsäureisopropylester, Essigsäureisobutylester und γ-Butrolacton; Kohlenwasserstoffe, z. B. Hexan, Heptan, Toluol, und Xylol; und Ketone, z. B. Isobutylmethylketon, Diethylketon und Isophoron.

Durch Zusatz eines der genannten Lösungsmittels kann die einzusetzende Menge an Acylierungsmittel reduziert werden. Das erfindungsgemäß verwendete Molverhältnis zwischen Acylierungsmittel und Endion liegt dann bevorzugt zwischen 2 : 1 und 3 : 1.

### Durchführung

### Beispiel 1:

Zu einer Suspension von 51,1 g (0,5 Mol) Acetanhydrid und 6,2 g H-Y-Zeolith (Modul SiO₂/Al₂O₃ = 25±5, 2 h bei 400°C aktiviert) wurden 15,2 g (0,1 Mol) 2,6,6-Trimethylcyclohex-2-en-1,4-dion bei 30 bis 40°C zugegeben. Diese Mischung wurde 5 h bei 60 bis 100°C gerührt, wobei die Reaktionsfortschritt per HPLC verfolgt wurde. Nach beendeter Reaktion wurde auf Raumtemperatur abgekühlt und der Katalysator durch Filtration abgetrennt. Das Filtrat, welches aus Essigsäure, nicht umgesetztem Acetanhydrid sowie gelöstem Trimethylhydrochinon-Diacetat besteht, wurde unter vermindertem Druck bei 60°C am Rotavapor zur Trockne eingeengt. Der Rückstand wurde in 150 ml Wasser aufgenommen, in einer Reibschale homogenisiert und der pH-Wert der Suspension mit Natronlauge auf 5-6 eingestellt. Das dabei erhaltene Trimethylhydrochinon-Diacetat wurde abgesaugt,mit Wasser gewaschen und im Vakuum getrocknet. Die Ausbeute betrug 22,5 g, entsprechend 95 % der Theorie.

### Beispiel 2:

7,7 g H-Y-Zeolith (Modul SiO₂/Al₂O₃ = 25±5, 2,5 h bei 450°C aktiviert) wurden unter Rühren in 50 ml Toluol suspendiert, und mit 30,6 g (0,3 Mol) Acetanhydrid und 15,2 g (0,1 Mol) 2,6,6-Trimethylcyclohex-2-en-1,4-dion 7 h bei 90 bis 110°C gerührt. Nach beendeter Reaktion wurde der Katalysator abfiltriert und mit Toluol gewaschen. Das Filtrat wurde am Rotavapor unter vermindertem Druck bei 60°C zur Trockne eingeengt. Der Rückstand wurde in 20 ml Essigsäure gelöst und zu 100 ml Wasser gegeben. Der pH-Wert der Suspension wurde mit Natronlauge auf 6 eingestellt. Das ausgefallene Trimethylhydrochinon-Diacetat wurde abgesaugt, mit Wasser gewaschen und im Vakuum getrocknet. Die Ausbeute betrug 22,4 g, entsprechend 95 % der Theorie.

### Beispiel 3:

Analog zu Beispiel 2 wurde anstelle von Toluol n-Propylacetat als Lösungsmittel eingesetzt. Die Ausbeute betrug 22,3 g, entsprechend 95 % der Theorie.

### Beispiel 4:

Zu einer Suspension von 7,8 g H-Y-Zeolith (Modul SiO₂/Al₂O₃ = 120 ± 20, 1 h bei 450°C aktiviert) und 76,6 g (0,75 Mol) Acetanhydrid wurden 15.2 g (0,1 Mol) 2,6,6-Trimethylcyclohex-2-en-1,4-dion rasch zugesetzt. Die Mischung wurde auf 95°C erwärmt und 3 h gerührt. Es wurde auf Raumtemperatur abgekühlt und analog zu Beispiel 1 aufgearbeitet. Die Ausbeute betrug 23,0 g, entsprechend 97 % der Theorie.

### Beispiel 5:

In analoger Weise zu Beispiel 4 wurden 7,4 g H-Y-Zeolith (Modul SiO₂/Al₂O₃ = 55 ± 10, 1 h bei 450°C aktiviert) in 76,6 g (0,75 Mol) Acetanhydrid suspendiert und 15,2 g (0,1 Mol) 2,6,6-Trimethylcyclohex-2-en-1,4-dion rasch zugesetzt. Nach 3 h Reaktion bei 30 bis 90°C und Aufarbeitung wie in Beispiel 1 wurden 23,0 g, entsprechend 97 % der Theorie.

### Beispiel 6:

10,0 g H-Beta-Zeolith (Modul SiO₂/Al₂O₃ = 27, 2 h bei 150°C aktiviert) wurden in 30,7 g (0,3 Mol) Acetanhydrid suspendiert und 15,3 g (0,1 Mol) 2,6,6-Trimethylcyclohex-2-en-1,4-dion zugesetzt. Das Gemisch wurde bei 140°C für 48 h gerührt und anschließend wie in Beispiel 1 aufgearbeitet. Die Ausbeute betrug 22,1 g, entsprechend 94 % der Theorie.

### Vergleichsbeispiel 7:

15,5 g MCM-41 (Modul SiO₂/Al₂O₃ = 25, 1h bei 150°C aktiviert) wurden in 76,6 g (0,75 Mol) Acetanhydrid suspendiert und 15,3 g (0,1 Mol) 2,6,6-Trimethylcyclohex-2-en-1,4-dion zugesetzt. Das Gemisch wurde bei 140°C für 21 h gerührt und anschließend wie in Beispiel 1 aufgearbeitet. Die Ausbeute betrug 20,4 g, entsprechend 86 % der Theorie.

### Vergleichsbeispiel 8:

In eine gerührte Suspension aus 30,6 g (0,30 mol) Acetanhydrid und 1,52 g Nafion® NR50 (10 - 35 mesh ) wurden bei 50°C innerhalb von 15 min 15,2 g (0,1 mol) 2,6,6-Trimethyl-cyclohex-2-en-1,4-dion zugetropft. Die Suspension wurde 2 Stunden bei 50 und 3 Stunden bei 80°C gerührt. Der Katalysator wurde durch Filtration abgetrennt und im Filtrat wurden mit HPLC 2,6,6-Trimethylcyclohex-2-en-1,4-dion und Trimethyl-hydrochinon-Diacetat bestimmt. Daraus ergibt sich ein Umsatz von 93,1% und eine Ausbeute von 85,1% der Theorie.

### Vergleichsbeispiel 9:

Als Katalysator wurde ein silikonvernetztes Nafion® eingesetzt, dessen Herstellung nach der Vorschrift in dem Artikel von Mark A. Harmer in *J. Am. Chem. Soc*., 118, *1996*, 7709, erfolgte. In eine Suspension aus 3,1 g dieses Katalysators und 30,6 g (0,30 mol) Acetanhyrid wurden bei 40°C innerhalb von 15 min 15,2 g (0,10 mol) 2,6,6-Trimethylcyclohex-2-en-1,4-dion eingerührt, wobei die Temperatur bis auf 51°C anstieg. Nach 5 Stunden bei 50°C betrug der Umsatz 96%. Nach der gleichen Aufarbeitung wie in Beispiel 1 beschrieben, wurden 22,1 g Produkt erhalten, was einer Ausbeute von 92,2 % der Theorie bedeutet.

### Vergleichsbeispiel 10:

Beispiel 9 wurde wiederholt, wobei die Katalysatormenge auf 4,5 g erhöht wurde. Nach einer Reaktionszeit von 3 Stunden bei 50°C betrug der Umsatz an 2,6,6-Trimethylcyclohex-2-en-1,4-dion 96,1%. Der Katalysator wurde abfiltriert, mit 10 ml Essigsäure gewaschen und zusammen mit 0,5 g frischem Katalysator erneut bei der Umlagerung eingesetzt. Nach einer Reaktionszeit von 3,5 Stunden betrug der Umsatz 95,8%.

## Patentansprüche

1. Verfahren zur Herstellung von 2,3,5-Trimethylhydrochinon-diestern durch Umsetzung von 2,6,6-Trimethylcyclohex-2-en-1,4-dion in Gegenwart eines Acylierungsmittels und einer Säure, **dadurch gekennzeichnet, daß** die Säure eine feste Säure ist und als Katalysator eingesetzt wird, wobei man den festen, sauren Katalysator ausgewählt aus der Gruppe HY- Zeolithe mit einem Modul zwischen 7,5 und 200, H-Beta-Zeolithe mit einem Modul zwischen 13 und 60, und H-Mordenite mit einem Modul zwischen 5 und 100 verwendet und die Umsetzung in flüssiger Phase bei einer Temperatur von 0 bis 140°C durchführt und den festen Katalysator anschließend abtrennt.

2. Verfahren gemäß Anspruch 1, **dadurch gekennzeichnet, dass** die als Katalysator eingesetzten Zeolithe einen Porendurchmesser von 0,5 bis 0,8 nm aufweisen.

3. Verfahren gemäß Anspruch 2, **dadurch gekennzeichnet, daß** man die Zeolithe der Gruppe HY-Zeolithe, H-Beta-Zeolithe oder H-Mordenite einzeln oder im Gemisch miteinander einsetzt.

4. Verfahren nach Ansprüchen 1 bis 3, **dadurch gekennzeichnet, daß** man den Katalysator in einer Menge von 5 bis 150 Gew.-% bezogen auf 2,6,6-Trimethylcyclohex-2-en-1,4-dion, insbesondere in einer Menge von 20 bis 60 % einsetzt.

5. Verfahren nach einem oder mehreren der vorhergehenden Ansprüche, **dadurch gekennzeichnet, daß** man als Acylierungsmittel ein Carbonsäureanhydrid der allgemeinen Formel 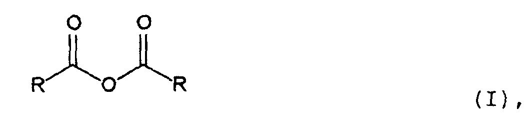 verwendet,
in der R einen gegebenenfalls substituierten aliphatischen, alicyclischen oder aromatischen Rest mit 1 bis 8 Kohlenstoffatomen bedeutet.

6. Verfahren nach einem oder mehreren der vorhergehenden Ansprüche, **dadurch gekennzeichnet, daß** man als Carbonsäureanhydrid Acetanhydrid verwendet.

7. Verfahren nach einem oder mehreren der vorhergehenden Ansprüche, **dadurch gekennzeichnet, daß** man als Acylierungsmittel ein Carbonsäurehalogenid verwendet.

8. Verfahren nach einem oder mehreren der vorhergehenden Ansprüche, **dadurch gekennzeichnet, daß** man als Acylierungsmittel einen Enolester der allgemeinen Formel 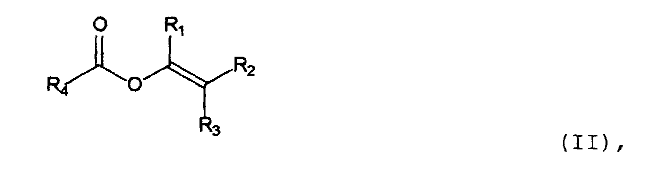 verwendet.
in der R₁ und R₂ Wasserstoffatome, Alkylreste mit 1 bis 8 Kohlenstoffatomen, oder Alkylenreste mit 1 bis 5 Kohlenstoffatomen, die gemeinsam einen 5- oder 6-gliedrigen Kohlenstoffring bilden, R₃ ein Wasserstoffatom oder einen Akylrest mit 1 bis 8 Kohlenstoffatomen und R₄ einen aliphatischen, acylischen oder aromatischen Rest mit 1 bis 8 Kohlenstoffatomen bedeuten.

9. Verfahren nach einen oder mehreren der vorgehenden Ansprüche, **dadurch gekennzeichnet, daß** man das Acylierungsmittel und das 2,6,6-Trimethyl-cyclohex-2-en-1,4-dion in einem Molverhältnis von 2 : 1 bis 20 : 1 verwendet, vorzugsweise 2 : 1 bis 5 : 1.

10. Verfahren nach einem oder mehreren der vorhergehenden Ansprüche, **dadurch gekennzeichnet, daß** man die Umsetzung bei einer Temperatur von 20 bis 100 °C durchführt.

11. Verfahren nach einem oder mehreren der vorhergehenden Ansprüche, **dadurch gekennzeichnet, daß** man die Umsetzung in Gegenwart eines organischen Lösungsmittels durchführt.

12. Verfahren nach einem oder mehreren der vorhergehenden Ansprüche, **dadurch gekennzeichnet, daß** man in Gegenwart eines organischen Lösungsmittels arbeitet und das Acylierungsmittel und das 2,6,6-Trimethylcyclo-2-en-1,4-dion in einem Molverhältnis von 2 : 1 bis 3 : 1 einsetzt.

13. Verfahren nach einem oder mehreren der vorhergehenden Ansprüche, **dadurch gekennzeichnet, daß** man den jeweils erhaltenen 2,3,5-Trimethylhydrochinondiester nach bekannten Maßnahmen zum 2,3,5-Trimethylhydrochinon verseift.

## Claims

1. Process for the preparation of 2,3,5-trimethylhydroquinone diesters by rearrangement of 2,6,6-trimethylcyclohex-2-ene-1,4-dione in the presence of an acylating agent and an acid, **characterised in that** the acid is a solid acid and is used as catalyst, wherein the solid, acid catalyst, selected from the group HY-zeolites with a modulus between 7.5 and 200, H-beta-zeolites with a modulus between 13 and 60, and H-mordenites with a modulus between 5 and 100, is used and the conversion is carried out in the liquid phase at a temperature from 0 to 140°C, and the solid catalyst is then separated.

2. Process according to claim 1, **characterised in that** the zeolites used as catalyst have a pore diameter of 0.5 to 0.8 nm.

3. Process according to claim 2, **characterised in that** the zeolites of the group HY-zeolites, H-beta-zeolites or H-mordenites are used individually or mixed with one another.

4. Process according to claims 1 to 3, **characterised in that** the catalyst is used in an amount of 5 to 150 wt.% referred to 2,6,6-trimethylcyclohex-2-ene-1,4-dione, especially in an amount of 20 to 60%.

5. Process according to one or more of the preceding claims, **characterised in that** as acylating agent a carboxylic acid anhydride of the general formula 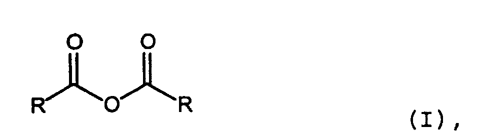 is used, in which R denotes an optionally substituted aliphatic, alicyclic or aromatic radical with 1 to 8 carbon atoms.

6. Process according to one or more of the preceding claims, **characterised in that** acetic anhydride is used as carboxylic acid anhydride.

7. Process according to one or more of the preceding claims, **characterised in that** a carboxylic acid halide is used as acylating agent.

8. Process according to one or more of the preceding claims, **characterised in that** as acylating agent an enol ester of the general formula 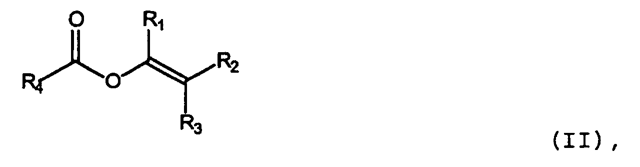 is used,
in which R₁ and R₂ denote hydrogen atoms, alkyl radicals with 1 to 8 carbon atoms, or alkylene radicals with 1 to 5 carbon atoms, which together form a 5- or 6-membered carbon ring, R₃ denotes a hydrogen atom or an alkyl radical with 1 to 8 carbon atoms and R₄ denotes an aliphatic, acyclic or aromatic radical with 1 to 8 carbon atoms.

9. Process according to one or more of the preceding claims, **characterised in that** the acylating agent and the 2,6,6-trimethylcyclohex-2-ene-1,4-dione are used in a molar ratio of 2:1 to 20:1, preferably 2:1 to 5:1.

10. Process according to one or more of the preceding claims, **characterised in that** the reaction is carried out at a temperature from 20 to 100°C.

11. Process according to one or more of the preceding claims, **characterised in that** the reaction is carried out in the presence of an organic solvent.

12. Process according to one or more of the preceding claims, **characterised in that** the reaction is carried out in the presence of an organic solvent and the acylating agent and the 2,6,6-trimethylcyclohex-2-ene-1,4-dione are used in a molar ratio of 2:1 to 3:1.

13. Process according to one or more of the preceding claims, **characterised in that** the 2,3,5-trimethylhydroquinone diester obtained in each case is saponified by known methods to form 2,3,5-trimethylhydroquinone.

## Revendications

1. Procédé de préparation de diesters de 2,3,5-triméthylhydroquinone par transformation de 2,6,6-triméthylcyclohex-2-ène-1,4-dione en présence d'un agent d'acylation et d'un acide, **caractérisé en ce que** l'acide est un acide solide et est utilisé comme catalyseur, en utilisant le catalyseur solide, acide choisi dans le groupe des zéolithes HY avec un module compris entre 7,5 et 200, des zéolithes H-bêta avec un module compris entre 13 et 60 et des mordénites H avec un module entre 5 et 100, on réalise la transformation en phase liquide à une température de 0 à 140°C et on sépare ensuite le catalyseur solide.

2. Procédé selon la revendication 1, **caractérisé en ce que** les zéolithes utilisées comme catalyseur présentent un diamètre de pores de 0,5 à 0,8 nm.

3. Procédé selon la revendication 2, **caractérisé en ce qu'**on utilise les zéolithes du groupe des zéolithes HY, des zéolithes H-bêta ou des mordénites H seules ou en mélange les unes avec les autres.

4. Procédé selon les revendications 1 à 3, **caractérisé en ce qu'**on utilise le catalyseur en une quantité de 5 à 150% en poids par rapport à la 2,6,6-triméthylcyclohex-2-ène-1,4-dione, en particulier en une quantité de 20 à 60%.

5. Procédé selon l'une ou plusieurs des revendications précédentes, **caractérisé en ce qu'**on utilise comme agent d'acylation un anhydride d'acide carboxylique de formule générale 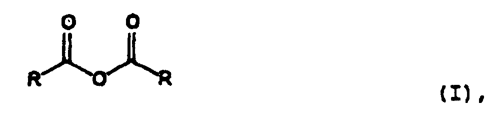 dans laquelle R signifie un radical aliphatique, alicyclique ou aromatique éventuellement substitué comprenant 1 à 8 atomes de carbone.

6. Procédé selon l'une ou plusieurs des revendications précédentes, **caractérisé en ce qu'**on utilise comme anhydride d'acide carboxylique l'anhydride de l'acide acétique.

7. Procédé selon l'une ou plusieurs des revendications précédentes, **caractérisé en ce qu'**on utilise comme agent d'acylation un halogénure d'acide carboxylique.

8. Procédé selon l'une ou plusieurs des revendications précédentes, **caractérisé en ce qu'**on utilise comme agent d'acylation un énolester de formule générale 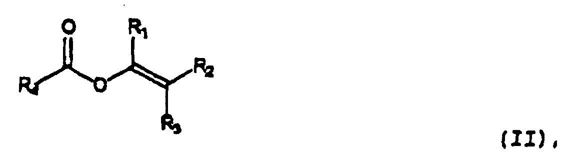 dans laquelle R₁ et R₂ signifient des atomes d'hydrogène, des radicaux alkyle comprenant 1 à 8 atomes de carbone ou des radicaux alkylène comprenant 1 à 5 atomes de carbone, qui forment ensemble un cycle hydrocarboné à 5 ou 6 chaînons, R₃ signifie un atome d'hydrogène ou un radical alkyle comprenant 1 à 8 atomes de carbone et R₄ signifie un radical aliphatique, acyclique ou aromatique comprenant 1 à 8 atomes de carbone.

9. Procédé selon l'une ou plusieurs des revendications précédentes, **caractérisé en ce qu'**on utilise l'agent d'acylation et la 2,6,6-triméthyl-cyclohex-2-ène-1,4-dione dans un rapport molaire de 2 : 1 à 20 : 1, de préférence de 2 : 1 à 5 : 1.

10. Procédé selon l'une ou plusieurs des revendications précédentes, **caractérisé en ce qu'**on réalise la transformation à une température de 20 à 100°C.

11. Procédé selon l'une ou plusieurs des revendications précédentes, **caractérisé en ce qu'**on réalise la transformation en présence d'un solvant organique.

12. Procédé selon l'une ou plusieurs des revendications précédentes, **caractérisé en ce qu'**on travaille en présence d'un solvant organique et on utilise l'agent d'acylation et la 2,6,6-triméthylcyclo-2-ène-1,4-dione dans un rapport molaire de 2 : 1 à 3 : 1.

13. Procédé selon l'une ou plusieurs des revendications précédentes, **caractérisé en ce qu'**on saponifie le diester de la 2,3,5-triméthylhydroquinone à chaque fois obtenu selon des procédures connues en 2,3,5-triméthylhydroquinone.
